# EUROPÄISCHE PATENTSCHRIFT

(11) **EP 1 358 930 B1**
(45) Veröffentlichungstag und Bekanntmachung des Hinweises auf die Patenterteilung: **02.10.2013**
(21) Anmeldenummer: 02009845.5
(22) Anmeldetag: 02.05.2002
(51) Int. Cl.: B01J 13/10

(54) **Mikrokapseln (XVII)**
Microcapsules (XVII)
Microcapsules (XVII)

(43) Veröffentlichungstag der Anmeldung: 05.11.2003
(73) Patentinhaber: Cognis IP Management GmbH, 40589 Düsseldorf (DE)
(72) Erfinder: Viladot Petit, Josep-Lluis, Dr., 08018 Barcelona (ES); De Moragas, Maria, Dr., 08310 Argentona (Barcelona) (ES)

(56) Entgegenhaltungen:
- EP-A- 0 379 379
- EP-A- 1 129 771
- FR-A- 2 748 673
- US-A- 5 051 304

## Beschreibung

### Gebiet der Erfindung

Die Erfindung befindet sich auf dem Gebiet der Verkapselung von Wirkstoffen und betrifft neue Mikrokapseln, ein Verfahren zur ihrer Herstellung sowie ihre Verwendung im Bereich der Kosmetik, Pharmazie und Lebensmittelzusatzstoffe.

### Stand der Technik

Unter dem Begriff "Mikrokapsel" werden sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden flüssige Wirkstoffe in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammem angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin **[**WO 01/01926**,** WO 01/01927**,** WO 01/01928**,** WO 01/01929**].**

Dokument EP-A-1 129 771 offenbart ein Verfahren zur Herstellung von Mikrokapseln mit mittlere Durchmessern im Bereich von 0,1 bis 0,5 mm, bei dem man (a) wässerige Wirkstoffzubereitungen mit Ölkörpern in Gegenwart von Emulgatoren zu O/W-Emulsionen verarbeitet, (b) die so erhaltenen Emulsionen mit wässrigen Lösungen anionischer Polymere behandelt, (c) die zo erhaltene Matrix mit wässrigen Chitosanlösungen in Kontakt bringt und (d) gegebenenfalls die so erhaltenen Verkapselungsprodukte von der wässrigen Phase abtrennt.

Die Freisetzung der Wirkstoffe aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung. Von Nachteil ist dabei, dass die Mikrokapseln die kontrollierte Freisetzung der Wirkstoffe aus ihrem Innern nicht oder nur in unzureichendem Maße zulassen und die Kapseln eine ungenügende Stabilität in Gegenwart von Tensiden, zumal anionischen Tensiden aufweisen. Die Aufgabe der vorliegenden Erfindung hat somit darin bestanden, gerade diese Nachteile zu überwinden.

### Beschreibung der Erfindung

Gegenstand der Erfindung ist ein Verfahren zur Herstellung von Mikrokapseln mit mittleren Durchmessern im Bereich von 0,001 bis 0,5, vorzugsweise 0,001 bis 0,1 und insbesondere 0,01 bis 0,05 mm, bei dem man
(a) wässrige Wirkstoffzubereitungen mit Ölkörpern in Gegenwart von Emulgatoren zu O/W-Emulsionen verarbeitet,
(b) die so erhaltenen Emulsionen mit wässrigen Lösungen anionischer Polymere behandelt,
(c) die so erhaltene Matrix mit wässrigen Lösungen nicht-biologischer kationischer Polymere in Kontakt bringt und
(d) gegebenenfalls die so erhaltenen Verkapselungsprodukte von der wässrigen Phase abtrennt.

Dabei entstehen Mikrokapseln durch Koazervation zwischen den anionischen und kationischen Polymeren, welche an den lipophilen Grenzflächen der O/W-Emulsionströpfchen stattfindet. Auf diese Weise gelingt es, auch solche Stoffe zu verkapseln, bei denen dies üblicherweise sehr schwierig ist. Zudem zeichnen sich die Mikrokapseln durch eine deutlich verbesserte Tensidstabilität aus.

### Wirkstoffe

Die Auswahl der zu verkapselnden Wirkstoffe ist an sich unkritisch. Es sollte sich um wasser- oder öllösliche Substanzen handeln, welche beispielsweise ausgewählt sein können aus der Gruppe der Fette und Wachse, Perlglanzwachse, Lecithine, Phospholipide, biogene Wirkstoffe, Enzyme, UV-Lichtschutzfaktoren, Antioxidantien, Deodorantien, Antitranspirantien, Antischuppenmittel, Filmbildner, Insektenrepellentien, Selbstbräuner, Tyrosininhibitoren, Parfümöle, Farbstoffe und dergleichen.

### ➢ Fette und Wachse

Typische Beispiele für Fette und Wachse sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-snglycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### ➢ Enzyme

Als zu verkapselnde Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt.

### ➢ UV-Lichtschutzfaktoren und Lichtschutzpigmente

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen: 3-Benzylidencampher bzw. 3-Benzylidennorcampher und dessen Derivate, z.B. 3-(4-Methylbenzyliden)campher; 4-Aminobenzoe-säurederivate, vorzugsweise 4-(Dimethyl-amino)benzoesäure-2-ethyl-hexylester, 4-(Dimethylamino)benzoesäure-2-octylester und 4-(Dimethylamino)benzoe-säureamyl-ester; Ester der Zimtsäure, vorzugsweise 4-Methoxyzimtsäure-2-ethylhexylester, 4-Methoxy-zimtsäurepropylester, 4-Methoxyzimtsäure-isoamylester 2-Cyano-3,3-phenyl-zimtsäure-2-ethylhexylester (Octocrylene); Ester der Salicylsäure, vorzugsweise Salicylsäure-2-ethylhexylester, Salicylsäure-4-iso-propylbenzylester, Salicylsäurehomomenthylester; Derivate des Benzophenons, vorzugsweise 2-Hydroxy-4-methoxybenzo-phenon, 2-Hydroxy-4-methoxy-4'-methylbenzophenon, 2,2'-Dihydroxy-4-methoxyben-zophenon; Ester der Benzalmalonsäure, vorzugsweise 4-Methoxybenzmalonsäuredi-2-ethylhexyl-ester; Triazinderivate, wie z.B. 2,4,6-Trianilino-(p-carbo-2'-ethyl-1'-hexyl-oxy)-1,3,5-triazin und Octyl Triazonoder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-De-rivate.

Als wasserlösliche Substanzen kommen in Frage: 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzo-phenon-5-sulfonsäure und ihre Salze; Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropyl-phenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimt-säureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### ➢ Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Typische Beispiele für geeignete Pflanzenextrakte sind die Wirkstoffe folgender Pflanzen : *Aesculus hippocastanum* (Rosskastanie), *Argania spinosa, Babtista tinctoria* (wilder Indigo), *Cantella asiatica, Camelilla sinensis* (Grüner Tee), *Chamonella recutita* (Kamille), *Ginkgo biloba* (Ginkgo), *Oleo europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna acontifolia*, und *Vitis vinifera* (wilder Wein).

Typische Beispiele für geeignete Antioxidantien sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### ➢ Deodorantien, keimhemmende Mittel und Enzyminhibitoren

Als adstringierende Deodorantien eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkoniumpentachlorohydrat und deren Komplexverbindungen z. B. mit Aminosäuren wie Glycin.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlor-phenol), 3-Methyl-4-(1-methylethyl)-phenol, 2-Benzyl-4-chlorphenol, 3-(4-Chlorphen-oxy)-1,2-propandiol, 3-Iod-2-propinylbutylcarbamat, Chlorhexidin, 3,4,4'-Trichlorcarb-anilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

### ➢ Weitere Wirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climba-zole), Ketoconazol®, (4-Acetyl-1-{-4-[2-(2.4-dichlorphenyl) r-2-(1H-imidazol-1-ylme-thyl)-1,3-dioxylan-c-4-ylmethoxyphenyl} piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachloro-phen), Undexylensäure Monoethanolamid Sulfosuccinat Na-Salz, Lamepon® UD (Protein-Undecylensäurekondensat), Zinkpyrithion, Aluminiumpyrithion und Magne-siumpyrithion / Dipyrithion-Magnesiumsulfat in Frage.

Als Insekten-Repellentien kommen N,N-Diethyl-m-toluamid, 1,2-Pentandiol oder Ethyl Butylacetylaminopropionate in Frage Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### ➢ Parfümöle

Als Parfümöle, die verkapselt werden können seien genannt : Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Alle genannten Wirkstoffe werden - bezogen auf die Mikrokapseln- üblicherweise in Konzentrationen von 0,001 bis 10, vorzugsweise 0,5 bis 5 und insbesondere 1 bis 2 Gew.-% eingesetzt.

### Ölkörper

Als Ölkörper, die Bestandteile der O/W-Emulsionen bilden, kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, E-rucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht. Die Menge der Ölkörper kann bezogen auf die Mikrokapseln 10 bis 30 und vorzugsweise 15 bis 30 Gew.-% betragen.

### Emulgatoren

Als Emulgatoren kommen grundsätzlich anionische, kationische oder ampholytische Tenside in Frage. Vorzugsweise werden jedoch nichtionogene Tenside eingesetzt, welche beispielsweise aus mindestens einer der folgenden Gruppen ausgewählt sein können.
➢ Anlagerungsprodukte von 2 bis 30 Mol Ethylenoxid und/ oder 0 bis 5 Mol Propylenoxid an lineare Fettalkohole mit 8 bis 22 Kohlenstoffatomen, an Fettsäuren mit 12 bis 22 Kohlenstoffatomen, an Alkylphenole mit 8 bis 15 Kohlenstoffatomen in der Alkylgruppe sowie Alkylamine mit 8 bis 22 Kohlenstoffatomen im Alkylrest;
➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandüsostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Die Konzentration der Emulgatoren kann bezogen auf die Mikrokapseln 1 bis 10 und vorzugsweise 2 bis 8 Gew.-% betragen.

### Anionpolymere

Als anionische Polymere eignen sich neben anionischen Polysacchariden, wie z.B. Carboxymethylcellulose, oder Poly(meth)acrylsäuren und deren Derivaten, wie z.B. Salzen und Estern, vorzugsweise Salze der Alginsäure. Bei der Alginsäure handelt es sich um ein Gemisch carboxylgruppenhaltiger Polysaccharide mit folgendem idealisierten Monomerbaustein:

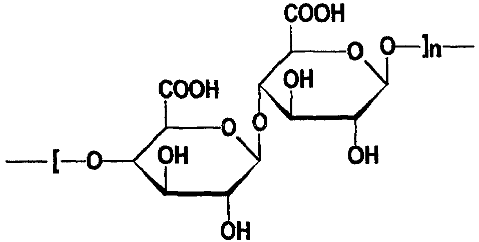

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Die Einsatzmenge der Anionpolymere beträgt in der Regel - bezogen auf die Mikrokapseln - 0,01 bis 1, vorzugsweise 0,05 bis 0,1 Gew.-%.

### Nicht-biologische Kationpolymere

Unter dem Begriff "nicht-biologische Kationpolymere" sind diejenigen kationaktiven Polymere zu verstehen, die auf synthetischem Wege erhalten werden, d.h. zu deren Herstellung nicht auf natürliche polymere Ausgangsstoffe, insbesondere nicht auf Chitin oder Chitosan zurückgegriffen werden muss; die erfindungsgemäßen Mikrokapseln sind vielmehr frei von diesen Stoffen. Typische Beispiele sind beispielsweise Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, Kondensationsprodukte aus Dihalogenalkylen, wie z.B. Dibrombutan mit Bisdialkylaminen, wie z.B. Bis-Dimethylamino-1,3-propan, sowie quaternierte Ammoniumsalz-Polymere, wie z.B. Mirapol® A-15, Mirapol® AD-1, Mirapol® AZ-1 der Firma Miranol. Die Einsatzmenge der Kationpolymere liegt - bezogen auf die Mikrokapseln - vorzugsweise im Bereich von 0,01 bis 1, vorzugsweise 0,05 bis 0,1 Gew.-%.

### Herstellung der Mikrokapseln

Zur Herstellung der erfindungsgemäßen Mikrokapseln wird zunächst eine O/W-Emulsion hergestellt, welche neben dem Ölkörper, Wasser und dem Wirkstoff eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Kationpolymerlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend können die Mikrokapseln von der wässrigen Phase beispielsweise durch Dekantieren, Filtrieren oder Zentrifugieren abgetrennt werden.

### Gewerbliche Anwendbarkeit

Ein weiterer Gegenstand der Erfindung betrifft die Verwendung der erfindungsgemäßen Mikrokapseln zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben. Diese können ihrerseits wieder Wirkstoffe, Ölkörper und Emulgatoren in unverkapselter Form enthalten, wie sie bereits eingangs beschrieben worden sind; auf eine erneute Aufzählung wird daher verzichtet. Bei den Zubereitungen kann es sich auch um Lebensmittelzusatzstoffe handeln, beispielsweise um Additive für Sportlernahrung und dergleichen. Die Mikrokapseln können in diesen Mitteln in Mengen von 1 bis 50, vorzugsweise 5 bis 15 Gew.-% enthalten sein.

### Beispiele

**Beispiel 1.** Im einer Rührapparatur wurden 0,5 g Konservierungsmittel (Phenonip®) in 50 g einer 2 Gew.-%igen wässrigen Zubereitung von Carboxymethylcellulose gelöst und die Mischung auf pH = 3,5 eingestellt. Anschließend wurde unter starkem Rühren eine Mischung bestehend aus 10 g einer 10 Gew.-%igen Lösung von Retinol in Sojaöl (Retinol® S20, BASF) und 0,5 g Sorbitanmonostearat+20EO (Eumulgin® SMS 20, Cognis Deutschland GmbH) hinzugegeben. Danach wurde unter weiterem Rühren eine solche Menge einer 10 Gew.-%igen Lösung von Polyquart 701/NA hinzugegeben, dass sich eine Kationpolymerkonzentration von 0,075 Gew.-% - bezogen auf die Zubereitung - einstellte. Schließlich wurde der pH-Wert durch Zugabe von Triethanolamin auf 5,5 angehoben und die entstandenen Mikrokapseln dekantiert.

**Beispiel 2. In** einer Rührapparatur wurden 0,5 g Konservierungsmittel (Phenonip®) in 50 g einer 2 Gew.-%igen wässrigen Zubereitung von Polyacrylsäure (Pemulen® TR-2) gelöst, wobei sich ein pH-Wert von 3 einstellte. Anschließend wurde unter starkem Rühren eine Mischung bestehend aus 10 g einer 10 Gew.-%igen Lösung von Retinol in Sojaöl (Retinol® S20, BASF) und 0,5 g Sorbitanmonolaurat+15EO (Eumulgin® SML 15, Cognis Deutschland GmbH) hinzugegeben. Danach wurde unter weiterem Rühren eine solche Menge einer 10 Gew.-%igen Lösung von Polyquart 701/NA hinzugegeben, dass sich eine Kationpolymerkonzentration von 0,01 Gew.-% - bezogen auf die Zubereitung - einstellte. Schließlich wurde der pH-Wert durch Zugabe von Triethanolamin auf 5,5 angehoben und die entstandenen Mikrokapseln dekantiert.

**Beispiel 3.** In einer Rührapparatur wurden 0,5 g Konservierungsmittel (Phenonip®) in 50 g einer 2 Gew.-%igen wässrigen Zubereitung von Polyacrylsäure (Pemulen® TR-2) gelöst, wobei sich ein pH-Wert von 3 einstellte. Anschließend wurde unter starkem Rühren eine Mischung bestehend aus 5 g Lösung von Tocopherol in Mineralöl und 0,5 g Coco Glucosides (Plantacare APG 1200, Cognis Deutschland GmbH) hinzugegeben. Danach wurde unter weiterem Rühren eine solche Menge einer 10 Gew.-%igen Lösung von Polyquart 701/NA hinzugegeben, dass sich eine Kationpolymerkonzentration von 0,075 Gew.-% - bezogen auf die Zubereitung - einstellte. Schließlich wurde der pH-Wert durch Zugabe von Triethanolamin auf 5,5 angehoben und die entstandenen Mikrokapseln dekantiert.

In Tabelle 1 finden sich eine Reihe von Formulierungsbeispielen.

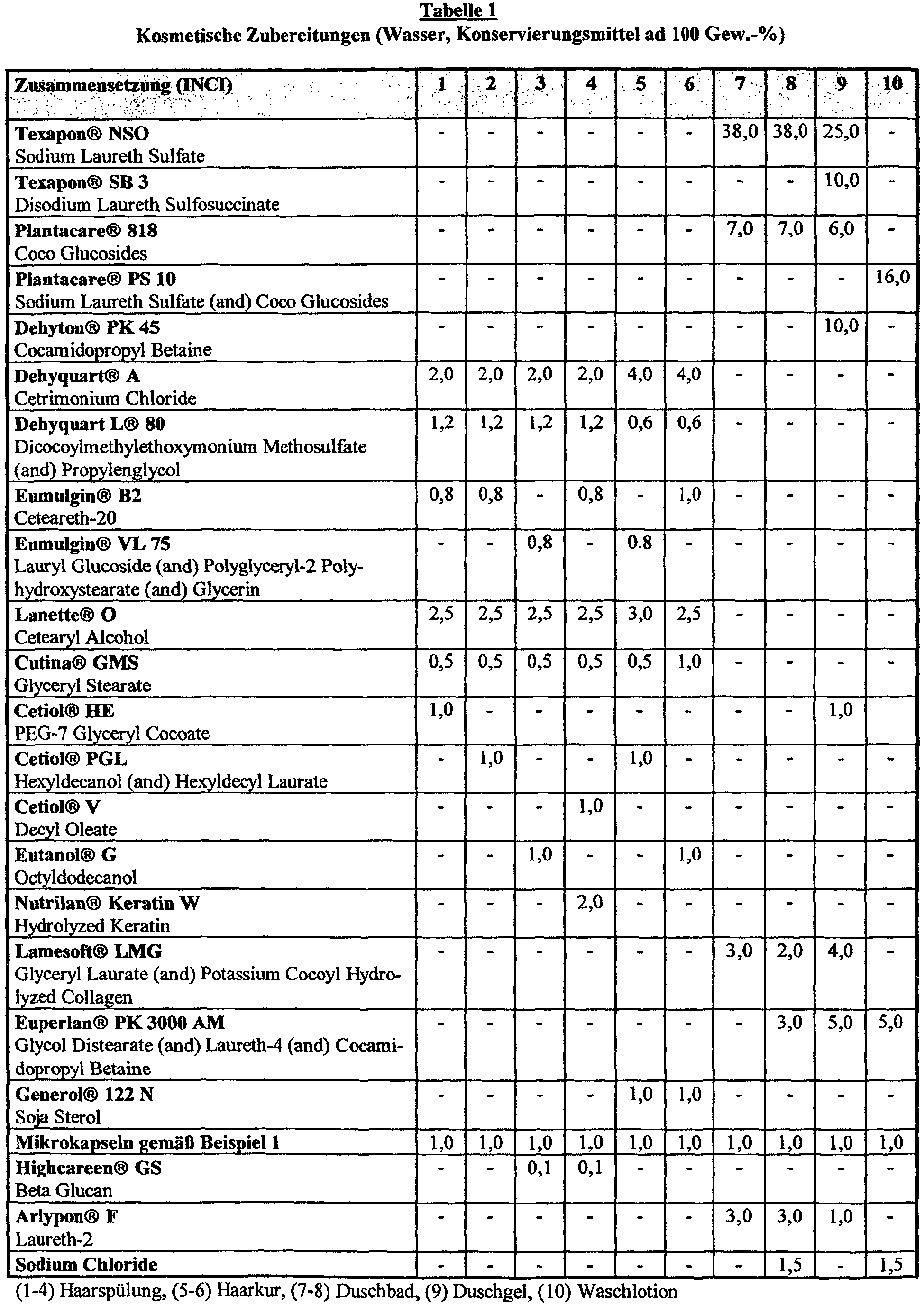

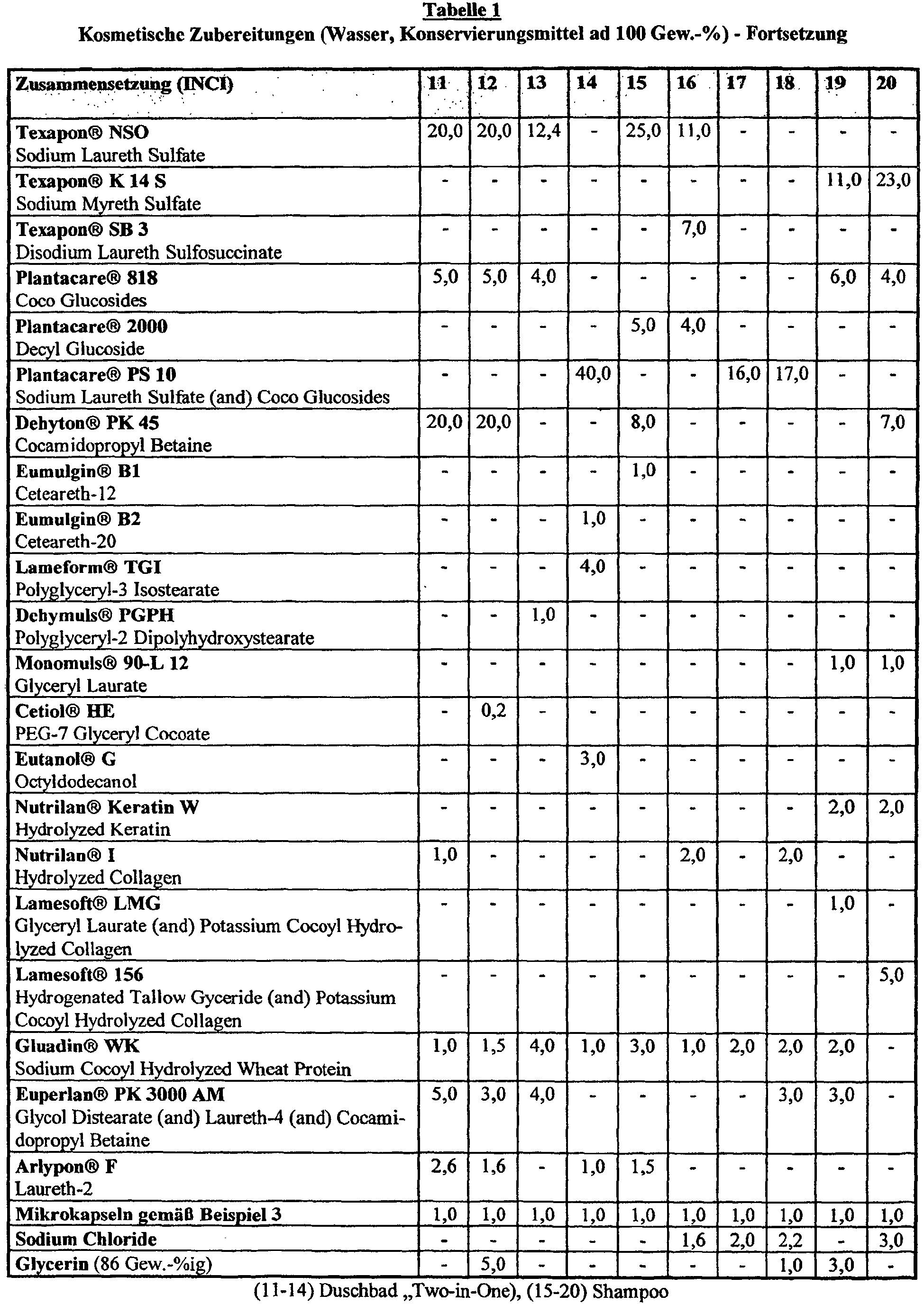

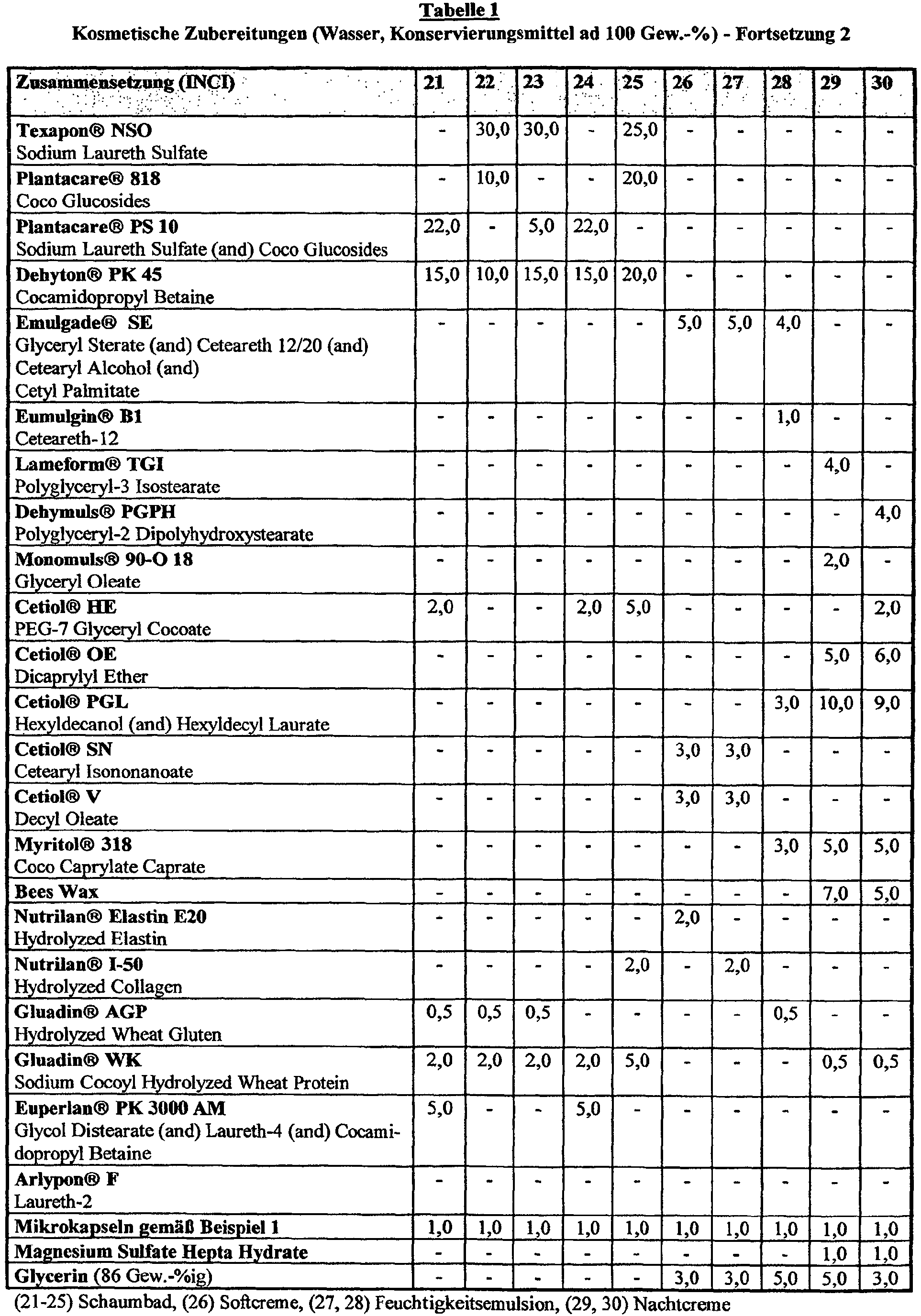

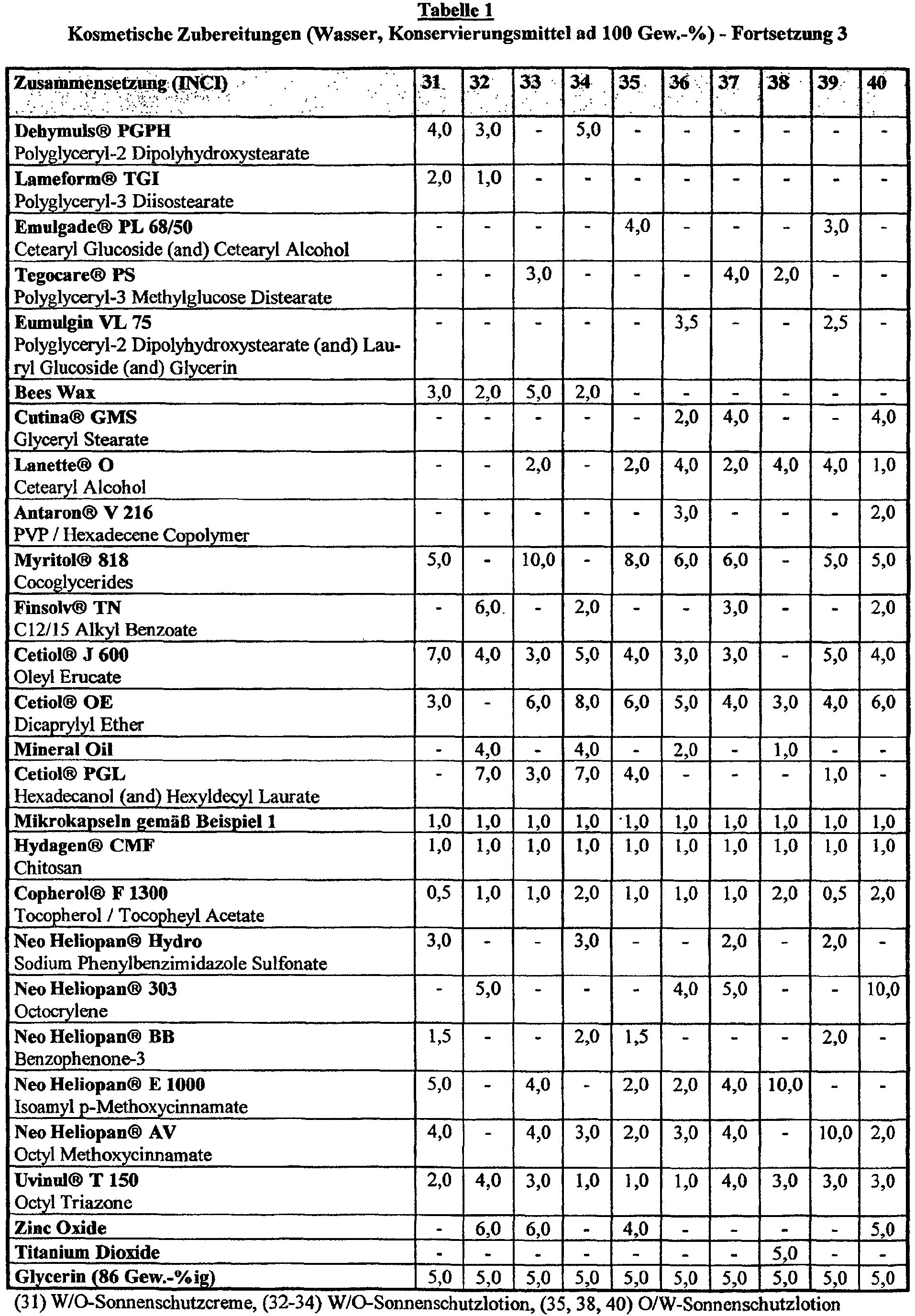

## Patentansprüche

1. Verfahren zur Herstellung von Mikrokapseln mit mittleren Durchmessern im Bereich von 0,0001 bis 0,5 mm, bei dem man
(a) wässrige Wirkstoffzubereitungen mit Ölkörpern in Gegenwart von Emulgatoren zu O/W-Emulsionen verarbeitet,
(b) die so erhaltenen Emulsionen mit wässrigen Lösungen anionischer Polymere behandelt,
(c) die so erhaltene Matrix mit wässrigen Lösungen nicht-biologischer kationischer Polymere in Kontakt bringt und
(d) gegebenenfalls die so erhaltenen Verkapselungsprodukte von der wässrigen Phase abtrennt.

2. Verfahren nach Anspruch 1, **dadurch gekennzeichnet, dass** man Wirkstoffe einsetzt, die ausgewählt sind aus der Gruppe, die gebildet wird von Fetten und Wachsen, Perlglanzwachsen, Lecithinen, Phospholipiden, biogenen Wirkstoffen, Enzymen, UV-Lichtschutzfaktoren, Antioxidantien, Deodorantien, Antitranspirantien, Antischuppenmitteln, Filmbildnern, Insektenrepellentien, Selbstbräunern, Tyrosininhibitoren, Parfümölen und Farbstoffen.

3. Verfahren nach den Ansprüchen 1 und/oder 2, **dadurch gekennzeichnet, dass** man Ölkörper einsetzt, die ausgewählt sind aus der Gruppe, die gebildet wird von Guerbetalkoholen auf Basis von Fettalkoholen mit 6 bis 18 Kohlenstoffatomen; Estern von linearen C₆-C₂₂-Fettsäuren mit linearen oder verzweigten C₆-C₂₂-Fettalkoholen; Estern von verzweigten C₆-C₁₃-Carbonsäuren mit linearen oder verzweigten C₆-C₂₂-Fettalkoholen; Estern von linearen C₆-C₂₂-Fettsäuren mit verzweigten Alkoholen; Estern von C₁₈-C₃₈-Alkylhydroxycarbonsäuren mit linearen oder verzweigten C₆-C₂₂-Fettalkoholen; Estern von linearen und/oder verzweigten Fettsäuren mit mehrwertigen Alkoholen und/oder Guerbetalkoholen; Triglyceriden auf Basis C₆-C₁₀-Fettsäuren; flüssigen Mono-/Di-/Triglyceridmischungen auf Basis von C₆-C₁₈-Fettsäuren; Estern von C₆-C₂₂-Fettalkoholen und/oder Guerbetalkoholen mit aromatischen Carbonsäuren; Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen; pflanzlichen Ölen; verzweigten primären Alkoholen; substituierten Cyclohexanen; linearen und verzweigte C₆-C₂₂-Fettalkoholcarbonaten; Guerbetcarbonaten auf Basis von Fettalkoholen mit 6 bis 18 Kohlenstoffatomen; Estern der Benzoesäure mit linearen und/oder verzweigten C₆-C₂₂-Alkoholen; linearen oder verzweigten, symmetrischen oder unsymmetrischen Dialkylethern mit 6 bis 22 Kohlenstoffatomen pro Alkylgruppe; Ringöffnungsprodukten von epoxidierten Fettsäureestern mit Polyolen; Siliconölen sowie aliphatischen bzw. naphthenischen Kohlenwasserstoffen und Mineralölen.

4. Verfahren nach mindestens einem der Ansprüche 1 bis 3, **dadurch gekennzeichnet, dass** man nichtionische Emulgatoren einsetzt.

5. Verfahren nach mindestens einem der Ansprüche 1 bis 4, **dadurch gekennzeichnet, dass** man nichtionische Emulgatoren einsetzt, die ausgewählt sind aus der Gruppe, die gebildet wird von Anlagerungsprodukten von 2 bis 30 Mol Ethylenoxid und/oder 0 bis 5 Mol Propylenoxid an lineare Fettalkohole mit 8 bis 22 Kohlenstoffatomen, an Fettsäuren mit 12 bis 22 Kohlenstoffatomen, an Alkylphenole mit 8 bis 15 Kohlenstoffatomen in der Alkylgruppe sowie Alkylamine mit 8 bis 22 Kohlenstoffatomen im Alkylrest; Alkyl- und/oder Alkenyloligoglykosiden mit 8 bis 22 Kohlenstoffatomen im Alk(en)ylrest und deren ethoxylierte Analoga; Anlagerungsprodukten von 1 bis 15 Mol Ethylenoxid an Ricinusöl und/oder gehärtetes Ricinusöl; Anlagerungsprodukten von 15 bis 60 Mol Ethylenoxid an Ricinusöl und/oder gehärtetes Ricinusöl; Partialestern von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid; Partialestern von Polyglycerin Polyethylenglycol, Trimethylolpropan, Pentaerythrit, Zuckeralkoholen Alkylglucosiden sowie Polyglucosiden mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid; Mischestern aus Pentaerythrit, Fettsäuren, Citronensäure und Fettalkohol und/oder Mischester von Fettsäuren mit 6 bis 22 Kohlenstoffatomen, Methylglucose und Polyolen; Mono-, Di- und Trialkylphosphaten sowie Mono-, Di- und/oder Tri-PEG-alkylphosphaten und deren Salzen; Wollwachsalkoholen; Polysiloxan-Polyalkyl-Polyether-Copolymeren; Block-Copolymeren; Polyalkylenglycolen sowie Glycerincarbonat.

6. Verfahren nach mindestens einem der Ansprüche 1 bis 5, **dadurch gekennzeichnet, dass** man anionische Polymere einsetzt, die ausgewählt sind aus der Gruppe, die gebildet wird von anionischen Polysacchariden, Poly(meth)acrylsäuren und deren Derivaten sowie Alginsäure und deren Salzen.

7. Verfahren nach mindestens einem der Ansprüche 1 bis 6, **dadurch gekennzeichnet, dass** man nicht-biologische kationische Polymere einsetzt, die ausgewählt sind aus der Gruppe, die gebildet wird von Copolymeren von Diallylammoniumsalzen und Acrylamiden, quaternierten Vinylpyrrolidon/Vinylimidazol-Polymeren, Kondensationsprodukten von Polyglycolen und Aminen, Polyethyleniminen, kationischen Siliconpolymeren, Copolymeren der Adipinsäure und Dimethylaminohydroxypropyldiethylentriamin, Copolymeren der Acrylsäure mit Dimethyldiallylammoniumchlorid, Polyaminopolyamiden sowie deren vernetzten wasserlöslichen Polymeren, Kondensationsprodukten aus Dihalogenalkylen und Bisdialkylaminen sowie quaternierten Ammoniumsalzpolymeren.

8. Verfahren nach mindestens einem der Ansprüche 1 bis 7, **dadurch gekennzeichnet, dass** die Mikrokapseln frei von Chitin und seinen Abbauprodukten Chitosan sind.

## Claims

1. A process for producing microcapsules having mean diameters in the range from 0.0001 to 0.5 mm, in which
(a) aqueous active compound preparations with oil droplets are processed in the presence of emulsifiers to give O/W emulsions,
(b) the resultant emulsions are treated with aqueous solutions of anionic polymers,
(c) the resultant matrix is contacted with aqueous solutions of non-biological cationic polymers and
(d) optionally the resultant encapsulated products are separated off from the aqueous phase.

2. The process according to claim 1, wherein active compounds are used which are selected from the group formed by fats and waxes, pearl gloss waxes, lecithins, phospholipids, biogenic active compounds, enzymes, UV-light protecting factors, antioxidants, deodorants, antitranspirants, antidandruff agents, film formers, insect repellants, self-tanning agents, tyrosine inhibitors, perfume oils and dyes.

3. The process according to claims 1 and/or 2, wherein oil droplets are used which are selected from the group formed by Guerbet alcohols based on fatty alcohols having 6 to 18 carbon atoms; esters of linear C₆-C₂₂ fatty acids with linear or branched C₆-C₂₂ fatty alcohols; esters of branched C₆-C₁₃ carboxylic acids with linear or branched C₆-C₂₂ fatty alcohols; esters of linear C₆-C₂₂ fatty acids with branched alcohols; esters of C₁₈-C₃₈ alkylhydroxycarboxylic acids with linear or branched C₆-C₂₂ fatty alcohols; esters of linear and/or branched fatty acids with polyhydric alcohols and/or Guerbet alcohols; triglycerides based on C₆-C₁₀ fatty acids; liquid mono-/di-/triglyceride mixtures based on C₆-C₁₈ fatty acids; esters of C₆-C₂₂ fatty alcohols and/or Guerbet alcohols with aromatic carboxylic acids; esters of C₂-C₁₂ dicarboxylic acids with linear or branched alcohols having 1 to 22 carbon atoms or polyols having 2 to 10 carbon atoms and 2 to 6 hydroxyl groups; vegetable oils; branched primary alcohols; substituted cyclohexanes; linear and branched C₆-C₂₂ fatty alcohol carbonates; Guerbet carbonates based on fatty alcohols having 6 to 18 carbon atoms; esters of benzoic acid with linear and/or branched C₆-C₂₂ alcohols; linear or branched, symmetrical or unsymmetrical dialkyl ethers having 6 to 22 carbon atoms per alkyl group; ring-opening products of epoxidized fatty acid esters with polyols; silicone oils and also aliphatic and/or naphthenic hydrocarbons and mineral oils.

4. The process according to at least one of claims 1 to 3, wherein nonionic emulsifiers are used.

5. The process according to at least one of claims 1 to 4, wherein nonionic emulsifiers are used which are selected from the group which is formed by addition products of 2 to 30 mol of ethylene oxide and/or 0 to 5 mol of propylene oxide to linear fatty alcohols having 8 to 22 carbon atoms, to fatty acids having 12 to 22 carbon atoms, to alkylphenols having 8 to 15 carbon atoms in the alkyl group and also alkylamines having 8 to 22 carbon atoms in the alkyl radical; alkyl- and/or alkenyloligoglycosides having 8 to 22 carbon atoms in the alk(en)yl radical and the ethoxylated analogs thereof; addition products of 1 to 15 mol of ethylene oxide to castor oil and/or hardened castor oil; addition products of 15 to 60 mol of ethylene oxide to castor oil and/or hardened castor oil; partial esters of glycerol and/or sorbitan with unsaturated linear or saturated, branched fatty acids having 12 to 22 carbon atoms and/or hydroxycarboxylic acids having 3 to 18 carbon atoms and/or adducts thereof with 1 to 30 mol of ethylene oxide; partial esters of polyglycerol polyethylene glycol, trimethylolpropane, pentaerythritol, sugar alcohols, alkyl-glucosides and also polyglucosides with saturated and/or unsaturated, linear or branched fatty acids having 12 to 22 carbon atoms and/or hydroxycarboxylic acids having 3 to 18 carbon atoms and also adducts thereof with 1 to 30 mol of ethylene oxide; mixed esters of pentaerythritol, fatty acids, citric acid and fatty alcohol and/or mixed esters of fatty acids having 6 to 22 carbon atoms, methylglucose and polyols; mono-, di- and trialkylphosphates and also mono-, di- and/or tri-PEG-alkylphosphates and salts thereof; lanolin alcohols; polysiloxane-polyalkyl-polyether copolymers; block copolymers; polyalkylene glycols and also glycerol carbonate.

6. The process according to at least one of claims 1 to 5, wherein anionic polymers are used which are selected from the group which is formed by anionic polysaccharides, poly(meth)acrylic acids and derivatives thereof and also alginic acid and salts thereof.

7. The process according to at least one of claims 1 to 6, wherein nonbiological cationic polymers are used which are selected from the group which is formed by copolymers of diallylammonium salts and acrylamides, quaternized vinylpyrrolidone/vinyl-imidazole polymers, condensation products of polyglycols and amines, polyethyleneimines, cationic silicone polymers, copolymers of adipic acid and dimethylaminohydroxypropyldiethylene-triamine, copolymers of acrylic acid with dimethyldiallylammonium chloride, polyaminopolyamides and also crosslinked water-soluble polymers thereof, condensation products of dihalo-alkyls and bisdialkylamines and also quaternized ammonium salt polymers.

8. The process according to at least one of claims 1 to 7, wherein the microcapsules are free from chitin and its breakdown product chitosan.

## Revendications

1. Procédé pour la préparation de microcapsules présentant des diamètres moyens dans la plage de 0,0001 à 0,5 mm, dans lequel
(a) on transforme des préparations aqueuses de substance active avec des corps huileux en présence d'émulsifiants en émulsions H/E,
(b) on traite les émulsions ainsi obtenues avec des solutions aqueuses de polymères anioniques,
(c) on met en contact la matrice ainsi obtenue avec des solutions aqueuses de polymères cationiques non biologiques et
(d) on sépare le cas échéant les produits d'encapsulation ainsi obtenus de la phase aqueuse.

2. Procédé selon la revendication 1, **caractérisé en ce qu'**on utilise des substances actives qui sont choisies dans le groupe formé par les graisses et les cires, les cires nacrées, les lécithines, les phospholipides, les substances actives biogènes, les enzymes, les facteurs de protection contre la lumière UV, les antioxydants, les déodorants, les antiperspirants, les agents antipelliculaires, les agents filmogènes, les répulsifs d'insectes, les autobronzants, les inhibiteurs de la tyrosine, les huiles parfumées et les colorants.

3. Procédé selon les revendications 1 et/ou 2, **caractérisé en ce qu'**on utilise des corps huileux qui sont choisis dans le groupe formé par les alcools de Guerbet à base d'alcools gras comprenant 6 à 18 atomes de carbone ; les esters d'acides gras linéaires en C₆-C₂₂ avec des alcools gras linéaires ou ramifiés en C₆-C₂₂ ; les esters diacides carboxyliques ramifiés en C₆-C₁₃ avec des alcools gras linéaires ou ramifiés en C₆-C₂₂ ; les esters d'acides gras linéaires en C₆-C₂₂ avec des alcools ramifiés ; des esters d'acides C₁₈-C₃₈-alkylhydroxycarboxyliques avec des alcools gras linéaires ou ramifiés en C₆-C₂₂ ; les esters d'acides gras linéaires et/ou ramifiés avec des alcools polyvalents et/ou des alcools de Guerbet ; les triglycérides à base d'acides gras en C₆-C₁₀; les mélanges liquides de monoglycérides/diglycérides/triglycérides à base diacides gras en C₆-C₁₈ ; les esters d'alcools gras en C₆-C₂₂ et/ou d'alcools de Guerbet avec des acides carboxyliques aromatiques; les esters d'acides C₂-C₁₂-dicarboxyliques avec des alcools linéaires ou ramifiés comprenant 1 à 22 atomes de carbone ou des polyols comprenant 2 à 10 atomes de carbone et 2 à 6 groupes hydroxyle ; les huiles végétales ; les alcools primaires ramifiés ; les cyclohexanes substitués ; les carbonates d'alcools gras linéaires et ramifiés en C₆-C₂₂ ; les carbonates de Guerbet à base d'alcools gras comprenant 6 à 18 atomes de carbone ; les esters de l'acide benzoïque avec des alcools en C₆-C₂₂ linéaires et/ou ramifiés ; les dialkyléthers linéaires ou ramifiés, symétriques ou asymétriques comprenant 6 à 22 atomes de carbone par groupe alkyle ; les produits d'ouverture de cycle d'esters d'acides gras époxydés avec des polyols ; les huiles de silicone ainsi que les hydrocarbures aliphatiques ou, selon le cas, naphténiques et les huiles minérales.

4. Procédé selon au moins l'une quelconque des revendications 1 à 3, **caractérisé en ce qu'**on utilise des émulsifiants non ioniques.

5. Procédé selon au moins l'une quelconque des revendications 1 à 4, **caractérisé en ce qu'**on utilise des émulsifiants non ioniques qui sont choisis dans le groupe formé par les produits d'addition de 2 à 30 moles d'oxyde d'éthylène et/ou 0 à 5 moles d'oxyde de propylène sur des alcools gras linéaires comprenant 8 à 22 atomes de carbone, sur des acides gras comprenant 12 à 22 atomes de carbone, sur des alkylphénols comprenant 8 à 15 atomes de carbone dans le groupe alkyle ainsi que des alkylamines comprenant 8 à 22 atomes de carbone dans le radical alkyle ; les alkyloligoglycosides et/ou les alcényloligoglycosides comprenant 8 à 22 atomes de carbone dans le radical alkyle/alcényle et leurs analogues éthoxylés ; les produits d'addition de 1 à 15 moles d'oxyde d'éthylène sur de l'huile de ricin et/ou de l'huile de ricin durcie ; les produits d'addition de 15 à 60 moles d'oxyde d'éthylène sur de l'huile de ricin et/ou de l'huile de ricin durcie ; les esters partiels de glycérol et/ou de sorbitane avec des acides gras insaturés, linéaires ou saturés, ramifiés comprenant 12 à 22 atomes de carbone et/ou les acides hydroxycarboxyliques comprenant 3 à 18 atomes de carbone ainsi que leurs produits d'addition de 1 à 30 moles d'oxyde d'éthylène ; les esters partiels de polyglycérol, de polyéthylèneglycol, de triméthylolpropane, de pentaérythritol, d'alcools de sucre, d'alkylglucosides ainsi que de polyglucosides avec des acides gras saturés et/ou insaturés, linéaires ou ramifiés comprenant 12 à 22 atomes de carbone et/ou des acides hydroxycarboxyliques comprenant 3 à 18 atomes de carbone ainsi que leurs produits d'addition de 1 à 30 moles d'oxyde d'éthylène; les esters mixtes de pentaérythritol, d'acides gras, d'acide citrique et d'alcool gras et/ou les esters mixtes d'acides gras comprenant 6 à 22 atomes de carbone, de méthylglucose et de polyols ; les monoalkylphosphates, les dialkylphosphates et les trialkylphosphates ainsi que les mono-PEG-alkylphosphates, les di-PEG-alkylphosphates et/ou les tri-PEG-alkylphosphates et leurs sels ; les alcools de cire de laine ; les copolymères de polysiloxane-polyalkyl-polyéther ; les copolymères à blocs ; les polyalkylèneglycols ainsi que le carbonate de glycérol.

6. Procédé selon au moins l'une quelconque des revendications 1 à 5, **caractérisé en ce qu'**on utilise des polymères anioniques qui sont choisis dans le groupe formé par les polysaccharides anioniques, les poly(acides (méth)acryliques) et leurs dérivés ainsi que l'acide alginique et ses sels.

7. Procédé selon au moins l'une quelconque des revendications 1 à 6, **caractérisé en ce qu'**on utilise des polymères cationiques non biologiques, qui sont choisis dans le groupe formé par les copolymères de sels de diallylammonium et d'acrylamides, les polymères quaternisés de vinylpyrrolidone/vinylimidazole, les produits de condensation de polyglycols et d'amines, les polyéthylène-imines, les polymères de silicone cationiques, les copolymères de l'acide adipique et de diméthylaminohydroxypropyldiéthylènetriamine, les copolymères de l'acide acrylique et de chlorure de diméthyldiallylammonium, les polyaminopolyamides ainsi que leurs polymères réticulés solubles dans l'eau, les produits de condensation de dihalogénoalkylène et de bisdialkylamines ainsi que les polymères quaternisés de sels d'ammonium.

8. Procédé selon au moins l'une quelconque des revendications 1 à 7, **caractérisé en ce que** les microcapsules sont exemptes de chitine et de ses produits de dégradation de type chitosane.
